# FASCICULE DE BREVET EUROPEEN

(11) **EP 2 239 004 B1**
(45) Date de publication et mention de la délivrance du brevet: **22.02.2012**
(21) Numéro de dépôt: 09305985.5
(22) Date de dépôt: 15.10.2009
(51) Int. Cl.: A61M 16/04

(54) **Dispositif d'assistance respiratoire et système de mesure comportant un tel dispositif**
Beatmungsunterstützungsgerät und ein solches beinhaltendes Messsystem
Respiratory aid device and measurement system comprising such a device

(30) Priorité: 09.04.2009 FR 0901752
(43) Date de publication de la demande: 13.10.2010
(73) Titulaire: Boussignac, Georges, 92160 Antony (FR)
(72) Inventeur: Boussignac, Georges, 92160 Antony (FR)
(74) Mandataire: Hauer, Bernard

(56) Documents cités:
- EP-A- 1 340 515
- US-A- 5 291 882
- US-A- 5 954 050
- US-A1- 2008 105 263

## Description

La présente invention a pour objet un dispositif d'assistance respiratoire utilisable sur des patients dont la respiratoire spontanée est absente ou insuffisante, qu'ils soient placés ou non sous respiration artificielle. Elle concerne de plus un système de mesure d'au moins un paramètre du gaz vicié expiré par les patients.

On connaît divers dispositifs d'assistance respiratoire, tels que des sondes, des canules orales, nasales, endotrachéales, trachéotomiques, destinées à faire la jonction entre un appareil de respiration artificiel et/ou d'anesthésie et le système respiratoire d'un patient.

Selon le cas, ces dispositifs comportent un tube qui forme un canal principal destiné à être relié, par son extrémité distale, à une voie respiratoire d'un patient pour que ce canal principal relie à l'extérieur le système respiratoire du patient, le dispositif comportant fréquemment au moins un canal auxiliaire, par exemple ménagé dans la paroi dudit tube, relié à une source de gaz respiratoire afin de permettre l'injection d'un jet de gaz respiratoire (oxygène, air ou mélange air-oxygène), continuel ou impulsionnel, destiné à la ventilation du patient, ce canal auxiliaire débouchant dans le canal principal au voisinage de l'extrémité distale de ce dernier.

Par ailleurs, on sait que, lors de l'utilisation d'un tel dispositif sur un patient en arrêt cardiaque, le redémarrage du coeur par massage cardiaque peut être détecté par une mesure de la pression partielle de gaz carbonique EtCO2 (pour en anglais « End-tidal CO2 » ou « CO2 télé-expiratoire » en français) dans l'air vicié expiré par le patient, au moyen d'un analyseur, par exemple un capnomètre. L'EtCO2 est un paramètre qui dépend de deux fonctions vitales importantes de l'organisme : la ventilation et la circulation sanguine, de sorte qu'il est un reflet simple de l'efficacité du massage cardiaque. Aussi, il est connu d'analyser l'air vicié expiré par le patient, en sortie de l'extrémité proximale du dispositif d'assistance respiratoire, de manière à déterminer la présence d'EtCO2 et ainsi détecter une éventuelle remise en marche du coeur du patient.

Un dispositif de ce genre est décrit dans US-5 291 882.

Cependant, l'air vicié sortant d'un tel dispositif respiratoire est généralement dilué par du gaz respiratoire, introduit dans le canal principal par l'intermédiaire du ou des canaux auxiliaires. Il est alors fréquent que la faible quantité d'EtCO2 présente dans l'air vicié, représentative de l'amorce du redémarrage du coeur, soit suffisamment diluée pour ne pas être détectée par le capnomètre. Ainsi, les opérateurs, manipulant le dispositif de respiration artificielle, ne sont avertis du redémarrage du coeur que lorsque la quantité d'EtCO2 du gaz vicié dilué dépasse un certain seuil de détection (le coeur ayant alors déjà redémarré depuis quelques instants). Ils poursuivent donc le massage cardiaque, formé de compressions et de décompressions alternées exercées sur la cage thoracique du patient, au moins jusqu'à ce qu'ils soient informés du redémarrage du coeur.

Or, s'il est prolongé dans le temps, un tel massage cardiaque engendre fréquemment, au niveau des poumons du patient, des lésions susceptibles de provoquer des écoulements de sang.

Aussi, il est préférable que le redémarrage du coeur soit signalé le plus tôt possible aux opérateurs, afin qu'ils cessent aussitôt de masser la cage thoracique du patient et qu'ils apportent d'autres soins plus appropriés.

La présente invention, telle que décrit dans la revendication 1 a pour objet de remédier à cet inconvénient et, notamment, de permettre une détection rapide du redémarrage du coeur d'un patient en état d'arrêt cardiaque.

Le dispositif d'assistance respiratoire comportant un tube qui forme un canal principal destiné à être relié, par son extrémité distale, à une voie respiratoire d'un patient pour que ledit canal principal relie à l'extérieur le système respiratoire dudit patient, ledit dispositif comportant au moins un canal auxiliaire relié à une source de gaz respiratoire pour pouvoir insuffler un jet d'un tel gaz respiratoire dans ledit système respiratoire et débouchant, par son extrémité distale, dans ledit canal principal au voisinage de l'extrémité distale de ce dernier, est remarquable en ce qu'il comporte des moyens pour prélever du gaz vicié, expiré par ledit patient, entre l'extrémité distale dudit canal auxiliaire et l'extrémité distale dudit canal principal.

Ainsi, grâce à l'invention, le gaz vicié expiré par le patient et prélevé par les moyens de prélèvement n'est pas, ou que très faiblement, dilué par du gaz respiratoire frais en provenance notamment du ou des canaux auxiliaires reliés à la source de gaz respiratoire. En conséquence, lorsque ces moyens de prélèvement sont reliés à des moyens de mesure de la pression partielle d'EtCO2 usuels (par exemple un capnomètre), il est possible de détecter et de mesurer de très faibles quantités d'EtCO2, qui seraient indécelables dans un air vicié dilué par du gaz respiratoire.

Dans le cas d'un patient en état d'arrêt cardiaque, les opérateurs peuvent ainsi être avertis immédiatement, ou quasiment immédiatement, de la remise en marche du coeur, ce qui leur permet de réagir rapidement et d'administrer au patient des soins appropriés.

Lesdits moyens de prélèvement de gaz vicié non dilué se présentent sous la forme d'au moins un canal de prélèvement. Ce dernier est ménagé dans l'épaisseur de la paroi dudit tube et s'étend sur au moins une partie de la longueur de ce dernier.

L'extrémité distale dudit canal de prélèvement débouche dans la face d'extrémité distale du tube, de sorte que le gaz vicié prélevé n'est pas, ou que très faiblement, dilué par du gaz respiratoire frais.

Par ailleurs, en regard de l'orifice distal dudit canal auxiliaire, des moyens de déflexion dudit jet de gaz respiratoire de ventilation vers l'axe dudit canal principal sont prévus.

L'invention, voir revendication 2, concerne également un système pour mesurer au moins un paramètre du gaz vicié expiré par une voie respiratoire d'un patient, ledit patient étant sous assistance respiratoire à l'aide d'un dispositif d'assistance respiratoire comportant un tube qui forme un canal principal destiné à être relié, par son extrémité distale, à ladite voie respiratoire du patient pour que ledit canal principal relie à l'extérieur le système respiratoire dudit patient, ledit dispositif comportant au moins un canal auxiliaire relié à une source de gaz respiratoire pour pouvoir insuffler un jet d'un tel gaz respiratoire dans ledit système respiratoire et débouchant, par son extrémité distale, dans ledit canal principal au voisinage de l'extrémité distale de ce dernier.

Selon l'invention, ledit dispositif comporte des moyens pour prélever du gaz vicié, expiré par le patient, entre l'extrémité distale dudit canal auxiliaire et l'extrémité distale dudit canal principal ; et ledit système comporte en outre des moyens de mesure dudit paramètre du gaz vicié expiré par le patient.

De plus, lesdits moyens de prélèvement de gaz vicié se présentent sous la forme d'au moins un canal de prélèvement ménagé dans l'épaisseur de la paroi dudit tube et s'étendant sur au moins une partie de la longueur de ce dernier, l'extrémité distale dudit canal de prélèvement débouchant dans la face d'extrémité distale du tube.

En outre, ledit système peut comporter un embout tubulaire creux destiné à être rapporté à l'extrémité proximale dudit tube, ledit embout tubulaire comportant un élément latéral saillant de raccord non communiquant avec l'espace intérieur dudit embout.

Par ailleurs, ledit système peut avantageusement comporter un raccord creux destiné à être rapporté, à une de ses extrémités, audit élément latéral saillant dudit embout tubulaire. En outre, ledit canal de prélèvement est destiné à être relié, par son extrémité proximale, à une autre extrémité dudit raccord. Ce dernier peut comporter un embout latéral, communiquant avec l'espace intérieur dudit raccord et destiné à être relié auxdits moyens de mesure.

De préférence, ledit paramètre est la pression partielle de gaz carbonique et lesdits moyens de mesure forment un capnomètre.
Les figures du dessin annexé feront bien comprendre comment l'invention peut être réalisée. Sur ces figures, des références identiques désignent des éléments semblables.
La figure 1 est une vue schématique partielle de profil d'un exemple de réalisation du dispositif d'assistance respiratoire conforme à la présente invention.
La figure 2 est une coupe schématique transversale du dispositif de la figure 1, selon la ligne II-II.
La figure 3 est une vue schématique partielle, en coupe axiale agrandie selon la ligne III-III de la figure 2, du dispositif d'assistance respiratoire de la présente invention.
La figure 4 montre, selon une vue semblable à la figure 3, un exemple de système de mesure conforme à la présente invention, mettant en oeuvre le dispositif d'assistance respiratoire de la figure 1.

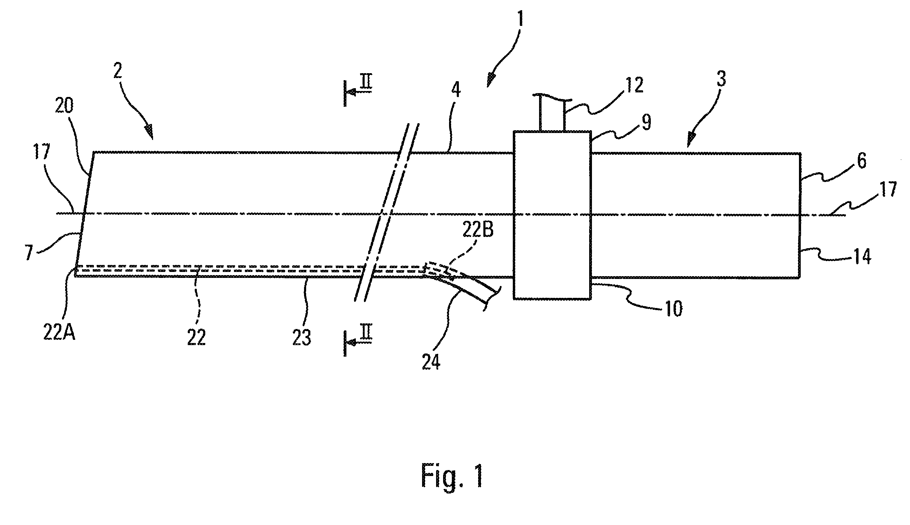

Sur la figure 1, on a représenté, schématiquement et à grande échelle, les seules portions proximale 2 et distale 3 d'un exemple de réalisation du dispositif d'assistance respiratoire 1 conforme à la présente invention. Ce dispositif 1 peut constituer, par exemple, une sonde endotrachéale oro-nasale avec ou sans ballonnet, une sonde endotrachéale pédiatrique, une sonde de monitorage des gaz, une sonde endobronchique, une sonde d'intubation anatomique pour enfant, une sonde de Cole néonatale, une sonde canule de Gedel, une sonde nasale d'oxygénothérapie, un masque nasal ou bucconasal ou un ballon nasal pour traitement d'apnée du sommeil.

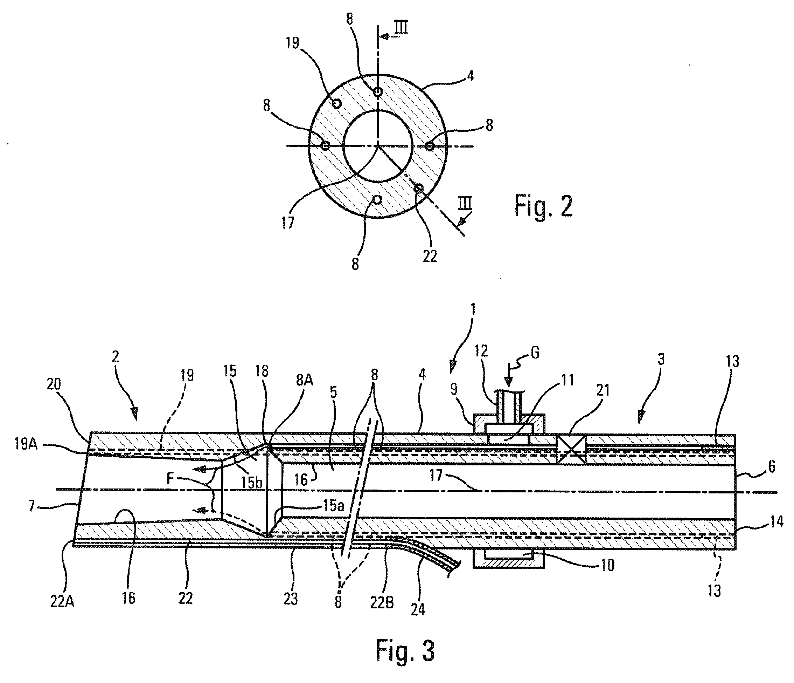

Comme le montrent les figures 2 et 3, le dispositif 1 comporte un tube 4, souple ou préformé (pour s'adapter à la morphologie du patient) délimitant un canal principal 5 ayant un orifice proximal 6 et un orifice distal 7, respectivement aux extrémités dudit tube 4.

Ainsi, le canal principal 5 est capable d'assurer le passage entre les orifices proximal 6 et distal 7, dont l'un (orifice distal 7) est destiné à se trouver à l'intérieur des voies respiratoires d'un patient et l'autre (orifice proximal 6) est destiné à se trouver à l'extérieur dudit patient. Cet orifice proximal 6 peut déboucher à l'air libre et, dans ce cas, le patient peut inspirer de l'air frais et expirer de l'air vicié à travers le canal principal 5. On peut également, comme cela est expliqué par la suite, relier l'orifice 6 à une source de gaz respiratoire sous pression et prévoir un système de valves unidirectionnelles, pour que le patient inspire le gaz respiratoire de ladite source à travers ledit canal principal 5 et expire le gaz vicié à l'air libre, également à travers ce canal principal 5.

Le diamètre du canal principal 5 est de l'ordre de quelques millimètres. Des essais satisfaisants ont été effectués avec des diamètres de 3 mm, 7 mm, 8 mm et 12 mm.

Par ailleurs, dans l'épaisseur de la paroi du tube 4, sont ménagés des canaux auxiliaires 8, s'étendant sur la presque totalité de la longueur du canal principal 5 et destinés à être reliés à une source de gaz respiratoire sous pression, comme cela est décrit ci-après.

La liaison à la source de gaz respiratoire peut être réalisée au moyen d'une bague 9, entourant de façon étanche le tube 4, du côté de la portion proximale 3 et délimitant une chambre annulaire étanche 10 autour dudit tube 4. Les canaux auxiliaires 8 sont mis en communication avec la chambre annulaire 10, grâce à des arrachements locaux 11 de la paroi du tube 4, et ladite chambre 10 est reliée à ladite source de gaz respiratoire par un conduit 12. Bien entendu, les extrémités proximales des canaux 8 sont obturées, par exemple par des bouchons 13, introduits à partir de la face d'extrémité proximale 14 du tube 4.

Les canaux auxiliaires 8 ont un diamètre plus petit que celui du canal principal 5. Le diamètre des canaux auxiliaires 8 est de préférence inférieur à 1 mm et, de façon avantageuse, il est de l'ordre de 5 à 800 microns. Du côté distal 8A, les canaux auxiliaires 8 débouchent dans un évidement 15 de la paroi interne 16 du tube 4. L'évidement 15 est annulaire et centré sur l'axe 17 dudit tube 4. Il comporte une face 15a, sensiblement transversale ou légèrement inclinée de façon à constituer un évasement du canal principal 5, dans laquelle débouchent lesdits canaux auxiliaires 8 par leurs orifices 18, ainsi qu'une face 15b suivant la face 15a et convergeant en direction de l'axe 17.

Ainsi, lorsque les canaux auxiliaires 8 sont alimentés en gaz respiratoire sous pression (flèche G sur la figure 3) à travers les éléments 9 à 12, les jets gazeux correspondants heurtent la face inclinée 15b, qui les défléchit en direction de l'axe 17 (flèche F sur la figure 3), engendrant au voisinage de celui-ci une zone de pression favorisant la circulation gazeuse à l'intérieur du canal principal 5, de l'orifice proximal 6 vers l'orifice distal 7. On favorise ainsi l'inspiration du patient.

Au moins un canal supplémentaire 19 est prévu dans l'épaisseur du tube 4 afin de déboucher en 19A au voisinage de la face d'extrémité distale 20 du tube 4 et servir de prise de pression.

A titre de sécurité, une soupape d'échappement tarée 21 peut être prévue au voisinage de la portion proximale 3 du tube 4. Ainsi, en cas de surpression accidentelle dans le canal principal 5, une fuite de gaz se produit à l'extérieur du patient, à travers la paroi du tube 4, pour éliminer instantanément cette surpression.

Comme le montre la figure 2, les canaux auxiliaires 8 sont disposés régulièrement autour de l'axe du tube 4. Leur nombre est variable suivant les utilisations (adulte ou enfant), mais il est généralement compris entre trois et neuf. De plus, au moins un des canaux auxiliaires 8 peut être spécialisé pour apporter un fluide médical.

Le tube 4 du dispositif 1 selon l'invention peut être réalisé en toute matière déjà utilisée dans les sondes respiratoires, par exemple en un chlorure de polyvinyle, avec un éventuel revêtement en silicone ou en acier permettant les injections à pression élevée.

Bien entendu, les dimensions du dispositif 1 selon l'invention peuvent être très variables, essentiellement en fonction de la voie de mise en place du tube et de la taille du patient, qui peut être un adulte, un enfant, un nourrisson ou un prématuré.

En outre, comme le montrent les figures 1 à 3, un canal de prélèvement 22 du gaz vicié, expiré par le patient et non dilué par le gaz respiratoire provenant des canaux auxiliaires 8, est ménagé dans l'épaisseur de la paroi du tube 4 et s'étend sur une partie de la longueur de ce dernier.

L'extrémité distale 22A du canal de prélèvement 22 débouche dans la face d'extrémité distale 20 du tube 4. Bien entendu, en variante, l'extrémité distale 22A du canal de prélèvement 22 pourrait déboucher dans la paroi externe 23 et/ou dans la paroi interne 16 du tube 4, entre l'extrémité distale 7 de ce dernier et l'orifice de sortie 18 des canaux auxiliaires 8.

Le canal de prélèvement 22 se prolonge par exemple à l'extérieur du tube 4, au moyen d'une tubulure 24 rapportée sur la paroi externe 23 dudit tube 4, au niveau de l'extrémité proximale 22B du canal 22, disposée en avant de la bague 9 entourant le tube 4.

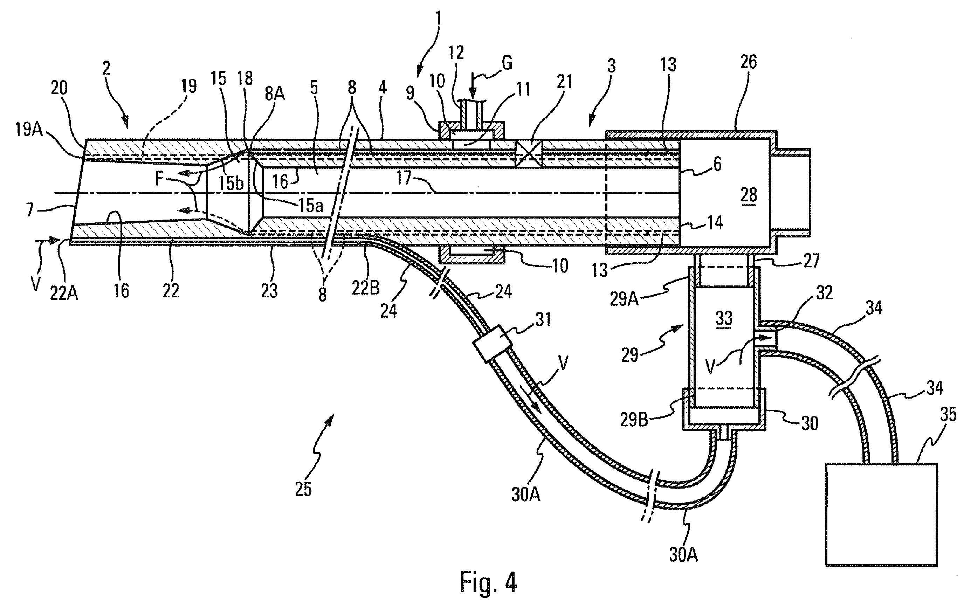

Par ailleurs, sur la figure 4, on a représenté un exemple de système de mesure 25 de la pression partielle de gaz carbonique EtCO2 conforme à l'invention, mettant en oeuvre le dispositif d'assistance respiratoire 1 précité (figures 1 à 3).

Un embout tubulaire creux 26 peut être rapporté, de façon amovible ou non, à l'extrémité proximale 6 du tube 4, de telle sorte qu'il prolonge ce dernier. Cet embout 26 comporte un élément latéral saillant de raccord 27, non communiquant avec l'espace intérieur 28 dudit embout 26.

Un raccord tubulaire creux 29 en forme de T, comportant deux extrémités en regard 29A et 29B, est apte à être relié à l'élément latéral 27 de l'embout 26 par l'extrémité 29A. L'autre extrémité 29B du raccord 29 peut être reliée, par exemple de façon amovible, à un embout de liaison 30, lui-même raccordé à un conduit 30A.

La tubulure 24 prolongeant le canal de prélèvement 22 du dispositif 1 peut être reliée, par des moyens de liaisons 31, au conduit 30A.

Le raccord tubulaire 29 comporte en outre un embout latéral 32 qui est en communication avec l'espace intérieur 33 dudit raccord 29. Il est apte à être relié, par l'intermédiaire d'un tube 34, à un dispositif de mesure 35 de la pression partielle de gaz carbonique expiré EtCO2 (par exemple un capnomètre).

Ainsi, le fonctionnement du système de mesure est le suivant. Le canal de prélèvement 22 prélève, à son extrémité distale 22A, du gaz vicié (flèche V) expiré par le patient, mais non dilué par du gaz respiratoire frais (flèche F). Ce gaz vicié non dilué prélevé est ensuite acheminé par l'intermédiaire du canal de prélèvement 22, de la tubulure 24, du conduit 30A, de l'espace intérieur 33 et du tube 34, au dispositif de mesure 35 pour être analysé.

## Revendications

1. Dispositif d'assistance respiratoire comportant un tube (4) qui forme un canal principal (5) destiné à être relié, par son extrémité distale (7), à une voie respiratoire d'un patient pour que ledit canal principal (5) relie à l'extérieur le système respiratoire dudit patient, ledit dispositif (1) comportant au moins un canal auxiliaire (8) relié à une source de gaz respiratoire pour pouvoir insuffler un jet d'un tel gaz respiratoire dans ledit système respiratoire et débouchant, par son extrémité distale (8A), dans ledit canal principal (5) au voisinage de l'extrémité distale (7) de ce dernier, ledit dispositif comportant au moins un canal (22) de prélèvement de gaz vicié non dilué ménagé dans l'épaisseur de la paroi dudit tube (4) et s'étendant sur au moins une partie de la longueur de ce dernier, des moyens de déflection (15) étant prévus en regard de l'orifice distal (18) dudit canal auxiliaire (8),
**caractérisé :**
- **en ce que** l'extrémité distale (22A) dudit canal de prélèvement (22) débouche dans la face d'extrémité distale (20) dudit tube (4) ; et
- **en ce que** lesdits moyens de déflexion (15) déflechissent le jet de gaz respiratoire de ventilation vers l'axe (17) dudit canal principal (5).

2. Système pour mesurer au moins un paramètre du gaz vicié expiré par une voie respiratoire d'un patient comportant un dispositif d'assistance respiratoire (1), ledit patient étant sous assistance respiratoire à l'aide dudit dispositif d'assistance respiratoire (1), ledit dispositif (1) comportant: un tube (4) qui forme un canal principal (5) destiné à être relié, par son extrémité distale (7), à ladite voie respiratoire du patient pour que ledit canal principal (5) relie à l'extérieur le système respiratoire dudit patient,
- au moins un canal auxiliaires (8) relié à une source de gaz respiratoire pour pouvoir insuffler un jet d'un tel gaz respiratoire dans ledit système respiratoire et débouchant, par son extrémité distale (8A), dans ledit canal principal (5) au voisinage de l'extrémité distale (7) de ce dernier ; et
- au moins un canal (22) de prélèvement de gaz vicié non dilué ménagé dans l'épaiseur de la paroi dudit tube (4) et s'étendant sur au moins une partie de la longueur de ce dernier,
**caractérisé :**
- **en ce que** l'extrétmité distale (22A) dudit canal de prélèvement (22) débouche dans la face d'extrémité distale (20) dudit tube (4) ;
- **en ce que** lesdits moyens de déflexion (15) déflechissent le jet de gaz respiratoire de ventilation vers l'axe (17) dudit canal principal (5) ; et
- **en ce que** ledit système (25) comporte en outre des moyens de mesure (35) dudit paramètre du gaz vicié expiré par le patient.

3. Système selon la revendication 2.
**caractérisé :**
- **en ce qu'**il comporte un embout tubulaire creux (26) destiné à être rapporté à l'extrémité proximale (6) dudit tube (4) ; et
- **en ce que** ledit embout tubulaire (26) comporte un élément latéral saillant de raccord (27), non communiquant avec l'espace intérieur (28) dudit embout (26).

4. Système selon la revendication 3,
**caractérisé :**
- **en ce qu'**il comporte un raccord creux (29) destiné à être rapporte, à une de ses extrémités (29A), audit élément latéral saillant (27) dudit embout tubulaire (26) ;
- **en ce que** ledit canal de prélèvement (22) est destiné à être relié, par son extrémité proximal (228), à une autre extrémité (29B) dudit raccord (29) .; et
- **en ce que** ledit raccord (29) comporte un embout latéral (32), communiquant avec l'espace intérieur (33) dudit raccord (29) et destiné à être relié auxdits moyens de mesure (35).

5. Système selon l'une des revendications 2 à 4,
**caractérisé:**
- **en ce que** ledit paramètre est la pression partielle de gaz carbonique ; et
- **en ce que** lesdits moyens de mesure (35) forment un capnomètre.

## Claims

1. A device for respiratory assistance, comprising a tube (4) which forms a main channel (5) designed to be connected, via its distal end (7), to an airway of a patient such that said main channel (5) connects the respiratory system of said patient to the outside, said device (1) comprising at least one auxiliary channel (8) which is connected to a source of respiratory gas, so as to be able to insufflate a jet of such a respiratory gas into said respiratory system, and which opens, via its distal end (8A), into said main channel (5) in the vicinity of the distal end (7) of the latter, said device comprising at least a removal channel (22) for undiluted vitiated gas formed within the thickness of the wall of said tube (4) and extending along at least part of the length of the latter, means (15) for deflecting being provided opposite the distal orifice (18) of said auxiliary channel (8),
**characterized in that**:
- the distal end (22A) of said removal channel (22) opens out into the distal end face (20) of said tube (4); and
- said means (15) for deflecting deflect the jet of respiratory ventilation gas toward the axis (17) of said main channel (5).

2. A system for measuring at least one parameter of the vitiated gas exhaled through an airway of a patient comprising a device for respiratory assistance (1), said patient being under respiratory assistance with the aid of said device (1) for respiratory assistance, said device (1) comprising:
- a tube (4) which forms a main channel (5) designed to be connected, via its distal end (7), to said airway of the patient such that said main channel (5) connects the respiratory system of said patient to the outside;
- at least one auxiliary channel (8) which is connected to a source of respiratory gas, so as to be able to insufflate a jet of such a respiratory gas into said respiratory system, and which opens, via its distal end (8A), into said main channel (5) in the vicinity of the distal end (7) of the latter; and
- at least a removal channel (22) for undiluted vitiated gas formed within the thickness of the wall of said tube (4) and extending along at least part of the length of the latter,
**characterized in that** :
- the distal end (22A) of said removal channel (22) opens out into the distal end face (20) of said tube (4);
- said means (15) for deflecting deflect the jet of respiratory ventilation gas toward the axis (17) of said main channel (5); and
- said system (25) additionally comprises means (35) for measuring said parameter of the vitiated gas exhaled by the patient.

3. The system as claimed in claim 2,
**characterized in that**:
- it comprises a hollow tubular endpiece (26) designed to be attached to the proximal end (6) of said tube (4); and
- said tubular endpiece (26) comprises a protruding lateral connector element (27) not communicating with the inner space (28) of said endpiece (26).

4. The system as claimed in claim 3,
**characterized in that**:
- it comprises a hollow connector (29) designed to be attached, at one of its ends (29A), to said protruding lateral element (27) of said tubular endpiece (26);
- said removal channel (22) is designed to be connected, via its proximal end (22B), to another end (29B) of said connector (29); and
- said connector (29) comprises a lateral nozzle (32) communicating with the inner space (33) of said connector (29) and designed to be connected to said measuring means (35).

5. The system as claimed in one of claims 2 to 4, **characterized in that**:
- said parameter is the partial pressure of carbon dioxide; and
- said measuring means (35) form a capnometer.

## Patentansprüche

1. Beatmungsgerät mit einer Röhre (4), die einen Hauptkanal (5) bildet, der dazu bestimmt ist, mit seinem distalen Ende (7) an einen Atemweg eines Patienten angeschlossen zu werden, damit der Hauptkanal (5) das Atemsystem des Patienten mit der freien Luft verbindet, wobei die Vorrichtung (1) wenigstens einen Hilfskanal (8) aufweist, der an eine Atemgasquelle angeschlossen ist, um einen Strahl eines derartigen Atemgases in das Atemsystem einblasen zu können, und der mit seinem distalen Ende (8A) in den Hauptkanal (5) in der Nähe des distalen Endes (7) desselben einmündet, wobei das Gerät wenigstens einen Entnahmekanal (22) für nicht verdünntes Abgas umfasst, der in der Dicke der Wand der Röhre (4) ausgebildet ist und sich über wenigstens einen Teil der Länge derselben erstreckt, wobei Ableitmittel (15) gegenüber der distalen Öffnung (18) des Hilfskanals (8) vorgesehen sind,
**dadurch gekennzeichnet:**
- **dass** das distale Ende (22A) des Entnahmekanals (22) in die distale Endseite (20) der Röhre (4) mündet; und
- **dass** die Ableitmittel (15) den Zulüftungs-Atemgasstrahl zur Achse (17) des Hauptkanals (5) hin ableiten.

2. System zur Messung von wenigstens einem Parameter des von einem Atemweg eines Patienten ausgeatmeten Abgases mit wenigstens einem Beatmungsgerät (1), wobei der Patient mithilfe des Beatmungsgeräts (1) künstlich beatmet wird, wobei das Gerät aufweist: eine Röhre (4), die einen Hauptkanal (5) bildet, der dazu bestimmt ist, mit seinem distalen Ende (7) mit dem Atemweg des Patienten verbunden zu werden, damit der Hauptkanal (5) das Atemsystem des Patienten mit der freien Luft verbindet:
- wenigstens einen Hilfskanal (8), der mit einer Atemgasquelle verbunden ist, um einen Strahl eines derartigen Atemgases in das Atemsystem einblasen zu können, und der mit seinem distalen Ende (8A) in den Hauptkanal (5) in der Nähe des distalen Endes (7) desselben einmündet; und
- wenigstens einen Entnahmekanal (22) für nicht verdünntes Abgas, der in der Dicke der Wand der Röhre (4) ausgebildet ist und sich über wenigstens einen Teil der Länge derselben erstreckt, **dadurch gekennzeichnet:**
- **dass** das distale Ende (22A) des Entnahmekanals (22) in die distale Endseite (20) der Röhre (4) mündet:
- **dass** die Ableitmittel (15) den Zulüftungs-Atemgasstrahl zur Achse (17) des Hauptkanals (5) hin ableiten; und
- **dass** das System (25) darüber hinaus Mittel zur Messung (35) des Parameters des vom Patienten ausgeatmeten Gases umfasst.

3. System nach Anspruch 2
**dadurch gekennzeichnet,**
- **dass** es einen hohlen röhrenförmigen Ansatz (26) aufweist, der dazu bestimmt ist, am proximalen Ende (6) der Röhre (4) aufgesetzt zu werden; und
- **dass** der röhrenförmige Ansatz (26) einen lateralen, hervorstehenden Anschluss (27) umfasst, der nicht mit dem inneren Raum (28) des Ansatzes (26) in Verbindung steht.

4. System nach Anspruch 3,
**dadurch gekennzeichnet,**
- **dass** es einen hohlen Anschluss (29) aufweist, der dazu bestimmt ist, an einem seiner Enden (29A) an das laterale, hervorstehende Element (27) des röhrenförmigen Ansatzes (26) aufgesetzt zu werden;
- **dass** der Entnahmekanal (22) dazu bestimmt ist, mit seinem proximalen Ende (22B) an ein anderes Ende (29B) des Anschlusses (29) angeschlossen zu werden; und
- **dass** der Anschluss (29) einen lateralen Ansatz (32) aufweist, der mit dem inneren Raum (33) des Anschlusses (29) in Verbindung steht und dazu bestimmt ist, mit den Messmitteln (35) verbunden zu werden.

5. System nach Anspruch 2 bis 4,
**dadurch gekennzeichnet:**
- **dass** der Parameter der Kohlendioxid-Teildruck ist; und
- **dass** die Messmittel (35) ein Kapnometer bilden.
